# EUROPÄISCHE PATENTANMELDUNG

(11) **EP 4 589 321 A1**
(43) Veröffentlichungstag der Anmeldung: **23.07.2025**
(21) Anmeldenummer: 24152289.5
(22) Anmeldetag: 17.01.2024
(51) Int. Cl.: G01R 33/38, G01R 33/385, G01R 33/36, G01R 33/54

(54) **MAGNETRESONANZGERÄT FÜR DEN TRANSPORT DURCH STANDARDISIERTE ZUGANGSWEGE**

(71) Anmelder: Siemens Healthineers AG, 91301 Forchheim (DE)
(72) Erfinder: Biber, Stephan, 91056 Erlangen (DE)
(74) Vertreter: Siemens Healthineers Patent Attorneys

(57) **Zusammenfassung**

Die Erfindung betrifft ein Magnetresonanzgerät (10), umfassend eine Haltestruktur (32) und eine Felderzeugungseinheit (11) mit einem Hauptmagneten (12), einem Gradientensystem und einem Hochfrequenzsystem, wobei die Haltestruktur (32) dazu ausgebildet ist, den Hauptmagneten (12) mechanisch zu stützen, und wobei die Felderzeugungseinheit (11) durch ein von der Haltestruktur (32) eingegrenztes Volumen außenumfänglich umschlossen ist.

## Beschreibung

Unabhängig vom grammatikalischen Geschlecht eines bestimmten Begriffes sind Personen mit männlicher, weiblicher oder anderer Geschlechteridentität mit umfasst.

Konventionelle Magnetresonanzgeräte für die medizinische Diagnostik wiegen mehrere Tonnen und besitzen Abmessungen in der Größenordnung von etwa 2 m Höhe und 1,6 m Länge. Solche Geräte lassen sich auf Grund dieser Eigenschaften nur an Orten, welche spezielle Anforderungen hinsichtlich Zugangs- und Transportmöglichkeiten sowie Tragkraft von Böden oder Decken erfüllen, installieren. Beispielsweise muss ein Untersuchungsraum, in welchem das Magnetresonanzgerät aufgestellt wird, eine ausreichend hohe Decke und entsprechend gestaltete Zugangswege besitzen. Weiterhin muss die Tragkraft der Decken des Untersuchungsraums das Gewicht des Magnetresonanzgeräts dauerhaft aushalten.

Insbesondere sind kleinere medizinische Einrichtungen und Praxen, welche eine sinnvolle Anwendung von Magnetresonanzbildgebungsverfahren in neuen Märkten (wie z. B. Zahnmedizin, Neurologie, Orthopädie) außerhalb der klassischen klinischen Radiologie versprechen, häufig nicht für die Aufstellung von konventionellen Magnetresonanzgeräten ausgestattet. Beispielsweise sind der Transport und die Aufstellung eines Magnetresonanzgeräts im laufenden Betrieb einer Zahnarztpraxis schwierig, da Kunden keine Unterbrechung des Betriebs durch Baustellen oder Umbauarbeiten, wie z. B. das Öffnen von Wänden oder Vergrößern von Türen, akzeptieren.

Für neue Märkte sind auch die Kosten eines Magnetresonanzgeräts, welche insbesondere durch die Länge eines in dem Hauptmagneten geformten Patientenaufnahmebereichs beeinflusst werden, sehr wichtig. Grundsätzlich wird der Hauptmagnet eines Magnetresonanzgeräts mit einem vorbestimmten Homogenitätsvolumen teurer, je kürzer er wird.

Darüber hinaus weisen konventionelle Magnetresonanzgeräte Teile auf, welche über den Hauptmagneten hinausstehen und fest mit diesem verbunden sind oder sich nicht leicht demontieren lassen (z. B. Teile der Körperspule und/oder der Gradientenspule, sowie Anschlüsse und Trägerstrukturen). Solche Teile vergrößern die Abmessung des Magnetresonanzgeräts und können den Transport sowie die Aufstellung des Magnetresonanzgeräts signifikant erschweren. Eine Verkürzung des Hauptmagneten übt sich bei gleichbleibenden Anforderungen an die Bildqualität negativ auf die Kosten des Hauptmagneten aus.

Es ist daher eine Aufgabe der Erfindung, ein Magnetresonanzgerät bereitzustellen, welches einen Transport durch standardisierte Zugangswege erlaubt, ohne eine Bildqualität zu reduzieren und/oder Kosten zu erhöhen.

Diese Aufgabe wird durch den Gegenstand des unabhängigen Patentanspruchs erfindungsgemäß gelöst. Vorteilhafte Ausführungsformen und zweckmäßige Weiterbildungen sind Gegenstand der Unteransprüche.

Das erfindungsgemäße Magnetresonanzgerät umfasst eine Haltestruktur und eine Felderzeugungseinheit mit einem Hauptmagneten, einem Gradientensystem und einem Hochfrequenzsystem.

Vorzugsweise ist das Magnetresonanzgerät dazu ausgebildet, eine Magnetresonanzmessung eines Objekts durchzuführen, welches innerhalb eines Bildaufnahmebereichs des Magnetresonanzgeräts positioniert ist. Das Magnetresonanzgerät kann beispielsweise dazu ausgebildet sein, Magnetresonanzdaten von dem Objekt zu erfassen, das innerhalb des Bildaufnahmebereichs ist. Ferner kann das Magnetresonanzgerät dazu ausgebildet sein, Magnetresonanzbilddaten, insbesondere diagnostische Magnetresonanzbilddaten, von dem innerhalb des Bildaufnahmebereichs positionierten Objekt zu erfassen. Das Objekt kann ein Patient sein, beispielsweise ein Mensch oder ein Tier.

Das Gradientensystem kann eine oder mehrere Gradientenspulen umfassen. Das Hochfrequenzsystem kann eine Hochfrequenzspule, insbesondere eine fest in das Magnetresonanzgerät integrierte Körperspule, umfassen. In einer bevorzugten Ausführungsform weisen die Gradientenspule(n) und die Hochfrequenzspule elektrische Leiterstrukturen auf, welche schalenförmig oder zylinderförmig ausgestaltet sind und den Bildaufnahmebereich des Magnetresonanzgeräts entlang einer Patientenzugangsrichtung umschließen. Es ist vorstellbar, dass die ein oder mehreren Gradientenspulen die Hochfrequenzspule entlang der Patientenzugangsrichtung umschließen.

In einer bevorzugen Ausführungsform ist das erfindungsgemäße Magnetresonanzgerät als ein geschlossener Scanner ("*closedbore scanner*") oder ein Scanner mit einem zylindrisch geformten Patiententunnel ausgestaltet. Ein geschlossener Scanner kann einen im Wesentlichen zylindrisch geformten Bildaufnahmebereich aufweisen. Der Hauptmagnet des geschlossenen Scanners kann eine oder mehrere Magnetspulen umfassen, welche den Bildaufnahmebereich entlang einer axialen Richtung oder einer Rotationsachse, insbesondere einer Rotationssymmetrie-Achse, des Hauptmagneten umschließen. Eine Magnetspule kann einen elektrischen Leiter mit vernachlässigbarem elektrischem Widerstand bei (oder unter) einer supraleitenden Temperatur umfassen. Eine Richtung eines Hauptmagnetfeldes, welches mittels des Hauptmagneten bereitgestellt wird, kann im Wesentlichen parallel zu einer Zugangsrichtung zu dem Bildaufnahmebereich und/oder der axialen Richtung der zylindrischen Bohrung ausgerichtet sein.

Es ist ebenso vorstellbar, dass das erfindungsgemäße Magnetresonanzgerät als ein offener Scanner ("open-bore scanner") ausgestaltet ist. Ein offener Scanner kann zwei Magnete umfassen, welche sich durch den Bildaufnahmebereich getrennt gegenüberstehen. Eine Richtung des Hauptmagnetfeldes des offenen Scanners kann im Wesentlichen orthogonal zu einer Zugangsrichtung zu dem Bildaufnahmebereich und/oder der axialen Richtung der zylindrischen Bohrung ausgerichtet sein. Ein von der Haltestruktur eingegrenztes Volumen kann bei einem offenen Scanner eine imaginäre Hülle darstellen, welche beide Magnete sowie die Haltestruktur, das Gradientensystem und das Hochfrequenzsystem mit einem kleinstmöglichen Volumen umschließt.

Der Hauptmagnet des Magnetresonanzgeräts kann einen oder mehrere Elektromagnete oder supraleitende Magnete umfassen oder daraus bestehen. In einer bevorzugten Ausführungsform umfasst oder besteht der Hauptmagnet aus einem oder mehreren zylindrisch geformten supraleitenden Magneten bzw. supraleitenden Spulen. Der Hauptmagnet kann mechanisch mit der Haltestruktur gekoppelt und/oder an der Haltestruktur befestigt sein. Vorzugsweise ist die Haltestruktur dazu ausgebildet, den Hauptmagneten zu tragen und/oder zu stützen. Der Begriff "Hauptmagnet" kann eine oder mehrere Magnete oder Spulen sowie eine dedizierte Stützstruktur für die Magnete oder Spulen umfassen.

Die hierin beschriebenen Konzepte lassen sich auch auf Hauptmagnete übertragen, welche Permanentmagnete umfassen oder aus Permanentmagneten bestehen.

Erfindungsgemäße ist die Haltestruktur dazu ausgebildet, den Hauptmagneten mechanisch zu stützen. Die Haltestruktur kann weiterhin dazu ausgebildet sein, den Hauptmagneten in einem von der Haltestruktur außenumfänglich umschlossenen Vakuumbereich einzuschließen.

Das erfindungsgemäße Magnetresonanzgerät kann eine äußere Vakuumkammer aufweisen. Die äußere Vakuumkammer kann einen Behälter darstellen, welcher für Fluide, insbesondere flüssige oder gasförmige Kryogene, undurchlässig ist. Die äußere Vakuumkammer kann insbesondere dazu ausgebildet sein, ein Vakuum in einem von der äußeren Vakuumkammer umschlossenen Vakuumbereich aufrechtzuerhalten. Vorzugsweise umschließt die äußere Vakuumkammer den Hauptmagneten in dem Vakuumbereich. Die äußere Vakuumkammer kann selbstverständlich weitere Komponenten des Magnetresonanzgeräts, wie z. B. einen Kryogenbehälter, eine thermische Abschirmung oder dergleichen, in dem Vakuumbereich einschließen.

Die äußere Vakuumkammer kann als ein Teil der Haltestruktur des Hauptmagneten ausgestaltet sein. Vorzugsweise ist der Hauptmagnet mittels dedizierter Befestigungselemente mechanisch mit der äußeren Vakuumkammer gekoppelt oder an dieser befestigt. Es ist vorstellbar, dass die äußere Vakuumkammer eine äußere Form der Haltestruktur und/oder des von der Haltestruktur eingegrenzten Volumens definiert oder vorgibt.

Vorzugsweise umfasst die äußere Vakuumkammer eine äußere Wand, eine innere Wand und Stirnwände, welche die äußere Wand und die innere Wand mechanisch verbinden. Die äußere Wand und die innere Wand können schalenförmig oder zylinderförmig ausgestaltet sein. Insbesondere kann die äußere Vakuumkammer als ein doppelwandiger Hohlzylinder ausgestaltet sein, welcher den Hauptmagneten in dem Vakuumbereich zwischen der äußeren Wand, der inneren Wand und ringförmigen Stirnwänden umschließt. Eine Zylinderachse der äußeren Vakuumkammer kann parallel zu einer Zylinderachse des Hauptmagneten ausgerichtet sein oder der Zylinderachse des Hauptmagneten entsprechen. Die innere Wand der äußeren Vakuumkammer kann einer Wand des Patiententunnels des Magnetresonanzgeräts entsprechen. Insbesondere können das Gradientensystem und/oder das Hochfrequenzsystem an der inneren Wand der äußeren Vakuumkammer befestigt sein und/oder von dieser getragen werden.

In einer bevorzugten Ausführungsform ist das erfindungsgemäße Magnetresonanzgerät als ein "trockenes" System ausgestaltet. Ein "trockenes" System kann eine geringe Menge an Kryogen oder gar kein Kryogen enthalten. Zum Beispiel kann das erfindungsgemäße Magnetresonanzgerät einen oder mehrere kleine Kryogenbehälter umfassen, welche mittels einer Wärmeleitstruktur thermisch mit dem Hauptmagneten verbunden sind. Ein Kryogenbehälter eines "trockenen" Systems kann ein Volumen von weniger als 10 l, weniger als 5 l oder weniger als 1 l an Kryogen enthalten. In einer Ausführungsform wird auf Kryogenbehälter verzichtet. In diesem Fall wird der Hauptmagnet vollständig mittels einer Wärmeleitstruktur gekühlt.

In einer alternativen Ausführungsform ist das Magnetresonanzgerät als ein "nasses" System ausgestaltet. Ein "nasses" System kann zumindest einen Kryogenbehälter mit einem Volumen von mehr als 10 l umfassen. Vorzugsweise ist der Hauptmagnet bei "nassen" Systemen innerhalb des Kryogenbehälters angeordnet und direkt durch das Kryogen gekühlt.

Ein Kryogen kann ein Fluid mit einem niedrigen Siedepunkt, wie z. B. Argon, Stickstoff, Neon, Helium oder dergleichen, darstellen. Eine Kühltemperatur des Kryogens kann im Wesentlichen einer supraleitenden Temperatur des Hauptmagneten entsprechen.

Es ist vorstellbar, dass Komponenten des Magnetresonanzgeräts, wie z. B. der Hauptmagnet, der Kryogenbehälter, die thermische Abschirmung, thermisch mit einem Kryokühler verbunden sind. Vorzugsweise sind die Komponenten des Magnetresonanzgerätes mittels einer Wärmeleitstruktur (z. B. einem wärmeleitfähigen Feststoff oder einem wärmeleitfähigen Metall), eines Konvektionskreislaufs und/oder eines Wärmerohrs *("heatpipe")* thermisch mit dem Kryokühler verbunden oder gekoppelt.

Erfindungsgemäß ist die Felderzeugungseinheit durch ein von der Haltestruktur eingegrenztes Volumen außenumfänglich umschlossen.

Die Felderzeugungseinheit kann eine Mehrzahl von Komponenten, wie z. B. den Hauptmagneten, das Gradientensystem und das Hochfrequenzsystem, umfassen. Vorzugsweise umfasst die Felderzeugungseinheit auch mit dem Gradientensystem und/oder dem Hochfrequenzsystem verbundene Anschlusselemente und/oder Trägerstrukturen.

In einer Ausführungsform umfasst die Felderzeugungseinheit zumindest ein Anschlusselement und/oder eine Trägerstruktur, welche durch das von der Haltestruktur eingegrenzte Volumen außenumfänglich umschlossen ist.

Der Kryokühler wird im Rahmen dieser Erfindung als eigenständige Komponente betrachtet und zählt somit nicht zu den Komponenten der Felderzeugungseinheit.

Vorzugsweise ist die Felderzeugungseinheit von einem durch die Haltestruktur umschlossenen Volumen vollständig eingefasst.

Ein von der Haltestruktur eingegrenztes oder umschlossenes Volumen kann ein Volumen einer imaginären Hülle darstellen, welche die Haltestruktur außenumfänglich mit einem kleinstmöglichen Volumen umschließt. Eine Form des von der Haltestruktur eingegrenzten oder umschlossenen Volumens kann mit einer äußeren Gestalt der Haltestruktur übereinstimmen.

In einer Ausführungsform sind die Komponenten der Felderzeugungseinheit entlang einer Patientenzugangsrichtung von dem von der Haltestruktur eingegrenzten Volumen außenumfänglich umschlossen.

Vorzugsweise sind die Komponenten der Felderzeugungseinheit derart innerhalb des Magnetresonanzgeräts angeordnet, dass ein Überstehen oder Hinausragen eines Teils der Felderzeugungseinheit über die Haltestruktur in axialer Richtung des Hauptmagneten und/oder entlang einer Patientenzugangsrichtung vermieden wird.

Insbesondere können die Komponenten der Felderzeugungseinheit derart innerhalb des Magnetresonanzgeräts angeordnet sein, dass ein Überstehen oder Hinausragen eines Teils der Felderzeugungseinheit über ein Ende oder eine Seite der Haltestruktur vermieden wird.

In einer besonders bevorzugten Ausführungsform ist das Magnetresonanzgerät als ein geschlossener Scanner ausgestaltet. Eine Länge der Haltestruktur entlang der Zylinderachse des Hauptmagneten kann weniger als 80 cm, weniger als 79 cm, weniger als 78 cm oder weniger als 77 cm betragen, wobei eine Länge der Felderzeugungseinheit mit der Länge der Haltestruktur entlang der Zylinderachse des Hauptmagneten übereinstimmt oder diese unterschreitet. Vorzugsweise definiert die Haltestruktur eine für den Hauptmagneten zur Verfügung stehende Länge des Hauptmagneten entlang der Zylinderachse des Hauptmagneten.

Durch das Vermeiden von Teilen der Felderzeugungseinheit, welche über eine Abmessung oder ein Ende der Haltestruktur hinausragen, lässt sich eine Größe des Hauptmagneten und damit eine Qualität des homogenen Volumens des erfindungsgemä-βen Magnetresonanzgeräts auf vorteilhafte Weise für Zugangswege mit standardisierten Abmessungen, wie z. B. Flure und Türen, optimieren. Dadurch kann das Magnetresonanzgerät auch in kleineren medizinischen Einrichtungen und Praxen installiert werden und qualitativ hochwertige Bilddaten bereitstellen.

Insbesondere lässt sich durch ein erfindungsgemäßes Magnetresonanzgerät ein Verkürzen des Hauptmagneten zugunsten von überstehenden Anschlusselementen und/oder Trägerstrukturen der Felderzeugungseinheit vermeiden. Dadurch kann eine Länge des Hauptmagneten entlang der Patientenzugangsrichtung vorteilhaft vergrößert oder optimiert werden. Ferner lassen sich durch das erfindungsgemäße Magnetresonanzgerät zusätzliche Kosten, welche mit einer Verkürzung des Hauptmagneten bei gleichbleibender Qualität des Bildgebungsvolumens verbunden sind, auf vorteilhafte Weise vermeiden oder reduzieren. Das erfindungsgemäße Magnetresonanzgerät kann weiterhin eine Verbesserung oder Optimierung eines Verhältnisses zwischen Abmessung und Kosten des Hauptmagneten ermöglichen.

In einer Ausführungsform des erfindungsgemäßen Magnetresonanzgeräts, weist das Hochfrequenzsystem eine Trägerstruktur auf, welche dazu ausgebildet ist, eine Hochfrequenzspule des Hochfrequenzsystems mechanisch mit der Haltestruktur zu koppeln.

Die Trägerstruktur kann dazu ausgebildet sein, die Hochfrequenzspule des Hochfrequenzsystems zu tragen und/oder eine mechanische Unterstützung für die Hochfrequenzspule bereitzustellen. Es ist vorstellbar, dass die Trägerstruktur dazu ausgebildet ist, die Hochfrequenzspule an der Haltestruktur zu befestigen.

Die Trägerstruktur kann ein beliebiges geeignetes mechanisches Element, wie z. B. eine Strebe, eine Stange, ein Balken, ein Winkel, ein L-Element, ein T-Element, ein V-Element oder dergleichen, umfassen. Die Trägerstruktur kann mittels einer beliebigen geeigneten mechanischen Verbindung mit der Hochfrequenzspule und/oder der Haltestruktur befestigt sein. Eine geeignete mechanische Verbindung kann eine formschlüssige Verbindung, eine kraftschlüssige Verbindung und/oder eine stoffschlüssige Verbindung umfassen. Beispielsweise kann eine mechanische Verbindung zwischen der Trägerstruktur und der Hochfrequenzspule, aber auch eine mechanische Verbindung zwischen der Trägerstruktur und der Haltestruktur, eine Schraubverbindung, eine Bolzenverbindung, eine Klemmverbindung, und/oder eine Klebverbindung umfassen.

In einer Ausführungsform ist die Trägerstruktur des Hochfrequenzsystems in einer Aussparung in einer Gradientenspule des Gradientensystems aufgenommen oder durch die Aussparung in einer Gradientenspule des Gradientensystems hindurchgeführt. Es ist vorstellbar, dass die Hochfrequenzspule durch eine Gradientenspule des Gradientensystems von der Haltestruktur des Hauptmagneten getrennt oder beabstandet ist. Eine Aussparung in dem Gradientensystem kann z. B. ein Ausschnitt oder ein Loch in einer Gradientenspule darstellen. Die Trägerstruktur kann insbesondere durch die Aussparung in dem Gradientensystem hindurchragen und die Hochfrequenzspule mechanisch mit der Haltestruktur des Hauptmagneten verbinden. Es ist vorstellbar, dass die Aussparung in dem Gradientensystem in einer Ebene oder einer gekrümmten Fläche einer elektrischen Leiterstruktur einer Gradientenspule des Gradientensystems angeordnet ist. Die elektrische Leiterstruktur der Gradientenspule kann um die Aussparung herum angeordnet sein oder die Aussparung umsäumen.

Die Trägerstruktur ist durch das von der Haltestruktur eingegrenzte Volumen außenumfänglich umschlossen.

Vorzugsweise ist die Trägerstruktur vollständig von dem von der Haltestruktur eingegrenzten Volumen eingefasst, sodass ein Überstehen der Trägerstruktur über ein Ende oder eine Seite der Haltestruktur vermieden wird.

Durch das Bereitstellen eines erfindungsgemäßen Magnetresonanzgeräts lässt sich ein Überstehen oder Hinausragen einer Trägerstruktur des Hochfrequenzsystems über ein Ende der Haltestruktur des Hauptmagneten auf vorteilhafte Weise vermeiden. Dadurch kann eine Abmessung des Hauptmagneten entlang einer Patientenzugangsrichtung auf vorteilhafte Weise vergrö-βert oder optimiert werden.

In einer Ausführungsform des erfindungsgemäßen Magnetresonanzgeräts umfasst die Haltestruktur eine äußere Vakuumkammer, welche den Hauptmagneten außenumfänglich umschließt. Eine Wand der äußeren Vakuumkammer weist eine Vertiefung auf, wobei ein Abschnitt der Trägerstruktur des Hochfrequenzsystems zumindest teilweise in der Vertiefung der äußeren Vakuumkammer aufgenommen ist.

Die äußere Vakuumkammer kann gemäß einer oben beschriebenen Ausführungsform ausgestaltet sein.

Die Vertiefung in der Wand der äußeren Vakuumkammer kann eine Senkung, eine Mulde und/oder ein Ausschnitt im Material der Wand der äußeren Vakuumkammer darstellen. Vorzugsweise wird die Wand der äußeren Vakuumkammer nicht durch die Vertiefung durchbrochen.

Die Vertiefung in der Wand der äußeren Vakuumkammer kann ein Volumen bereitstellen, welches für die mechanische Verbindung mit der Trägerstruktur des Hochfrequenzsystems, aber auch einer Trägerstruktur des Gradientensystems genutzt wird. In einem Beispiel kann die Trägerstruktur des Hochfrequenzsystems, aber auch eine Trägerstruktur des Gradientensystems, in der Vertiefung in der Wand der äußeren Vakuumkammer mechanisch befestigt oder verankert sein.

In einer Ausführungsform des Magnetresonanzgeräts sind das Gradientensystem und das Hochfrequenzsystem mittels einer Trägerstruktur mechanisch mit der Haltestruktur verbunden und/oder an der Haltestruktur befestigt.

Durch das Bereitstellen einer Vertiefung in einer Wand der äußeren Vakuumkammer kann die Trägerstruktur des Hochfrequenzsystems mechanisch mit der Haltestruktur des Hauptmagneten verbunden werden. Dadurch lässt sich eine erforderliche Abmessung der Aussparung in dem Gradientensystem auf vorteilhafte Weise reduzieren oder minimieren.

In einer weiteren Ausführungsform des Magnetresonanzgeräts, weist das Hochfrequenzsystem eine provisorische Trägerstruktur auf. Die provisorische Trägerstruktur ist dazu ausgebildet, eine Hochfrequenzspule des Hochfrequenzsystems reversibel an einer Gradientenspule des Gradientensystems und/oder der Haltestruktur zu befestigen.

Die provisorische Trägerstruktur kann mittels einer beliebigen geeigneten kraftschlüssigen und/oder formschlüssigen mechanischen Verbindung mit dem Gradientensystem und/oder der Haltestruktur verbunden sein. Beispielweise kann die provisorische Trägerstruktur mittels einer Schraubverbindung, einer Klemmverbindung und/oder einer Bolzenverbindung mit der Gradientenspule und/oder der Haltestruktur verbunden sein. Die provisorische Trägerstruktur kann in vergleichbarer Weise mit der Hochfrequenzspule mechanisch verbunden sein. Vorzugsweise ist die provisorische Trägerstruktur dazu ausgebildet, die Hochfrequenzspule des Hochfrequenzsystems, und optional auch die Gradientenspule des Gradientensystems, vorübergehend oder temporär an der Haltestruktur zu befestigen. Die provisorische Trägerstruktur kann ein mechanisches Element gemäß der oben beschriebenen Trägerstruktur aufweisen. Die provisorische Trägerstruktur kann insbesondere als ein Transportbolzen oder eine Transportschraube ausgestaltet sein.

In einer Ausführungsform verbindet die provisorische Trägerstruktur die Hochfrequenzspule des Hochfrequenzsystems mechanisch mit der Gradientenspule des Gradientensystems, wobei das Gradientensystem mechanisch mit der Haltestruktur des Hauptmagneten verbunden ist.

In einer weiteren Ausführungsform ragt die provisorische Trägerstruktur durch eine Aussparung in der Gradientenspule durch das Gradientensystem hindurch und verbindet die Hochfrequenzspule des Hochfrequenzsystems, und optional die Gradientenspule des Gradientensystems, mit der Haltestruktur des Hauptmagneten.

Gemäß der Erfindung ist die provisorische Trägerstruktur reversibel entfernbar ausgestaltet und in einer anwendungsgemä-ßen Anordnung zur Befestigung der Hochfrequenzspule des Hochfrequenzsystems an der Gradientenspule des Gradientensystems und/oder der Haltestruktur durch das von der Haltestruktur eingegrenzte Volumen außenumfänglich umschlossen.

Es ist vorstellbar, dass die provisorische Trägerstruktur nach dem Transport des Magnetresonanzgeräts durch eine konventionelle Trägerstruktur oder eine Trägerstruktur gemäß einer oben beschriebenen Ausführungsform ersetzt wird.

Eine provisorische Trägerstruktur muss lediglich dazu ausgelegt sein, mechanischen Kräften, welche während des Transports des Magnetresonanzgeräts auftreten, standzuhalten. Dadurch können eine erforderliche mechanische Verankerung in der Haltestruktur des Hauptmagneten und/oder eine Aussparung in dem Gradientensystem auf vorteilhafte kleiner dimensioniert werden. Die provisorische Trägerstruktur kann nach dem Transport des Magnetresonanzgeräts durch eine konventionelle Trägerstruktur ersetzt werden, welche auch (elektromagnetischen) Kräften, welche im Betrieb des Magnetresonanzgeräts auftreten, standhalten kann.

In einer bevorzugten Ausführungsform des Magnetresonanzgeräts weist eine Hochfrequenzspule des Hochfrequenzsystems ein Anschlusselement auf, welches dazu ausgebildet ist, die Hochfrequenzspule mit einer Stromquelle und/oder einem externen Kühlsystem zu verbinden.

Das Anschlusselement der Hochfrequenzspule des Hochfrequenzsystems kann einen elektrischen Anschluss umfassen. Der elektrische Anschluss kann beispielsweise als ein Pin, eine Klemme, ein Stecker oder eine Buchse ausgeführt sein. Der elektrische Anschluss kann dazu ausgebildet sein, eine elektrische Leiterstruktur der Hochfrequenzspule an eine Stromquelle, insbesondere eine Hochfrequenzeinheit des Magnetresonanzgeräts, anzuschließen.

Das Anschlusselement der Hochfrequenzspule kann weiterhin einen Kühlanschluss umfassen. Ein Kühlanschluss kann z. B. eine Rohrverbindung, eine Schlauchverbindung oder einen Anschlussstutzen umfassen. Der Kühlanschluss kann dazu ausgebildet sein, einen Kühlkreislauf des Hochfrequenzsystems mit einem Kühlmittel zu versorgen und/oder den Kühlkreislauf des Hochfrequenzsystems thermisch mit einem externen Kühlkreislauf zu koppeln.

Gemäß der Erfindung ist das Anschlusselement der Hochfrequenzspule durch das von der Haltestruktur eingegrenzte Volumen außenumfänglich umschlossen.

In einer Ausführungsform ist das Anschlusselement der Hochfrequenzspule in der Vertiefung in der Wand der äußeren Vakuumkammer gemäß einer oben beschriebenen Ausführungsform angeordnet. Es ist jedoch ebenso vorstellbar, dass das Anschlusselement der Hochfrequenzspule in einer Aussparung in einer Gradientenspule des Gradientensystems angeordnet ist. Weiterhin kann das Anschlusselement der Hochfrequenzspule auch in einen Patientenaufnahmebereich des Magnetresonanzgeräts hineinragen.

Bei konventionellen Magnetresonanzgeräten stehen elektrische Anschlüsse und/oder Kühlanschlüsse typischerweise über ein Ende oder eine Grundfläche der äußeren Vakuumkammer hinaus, weshalb der Hauptmagnet bei einem Transport durch standardisierte Zugangswege entsprechend verkürzt werden muss. Durch das Bereitstellen einer Hochfrequenzspule mit einem erfindungsgemäßen Anschlusselement kann eine Abmessung des Hauptmagneten vorteilhaft vergrößert oder optimiert werden.

In einer weiteren Ausführungsform des erfindungsgemäßen Magnetresonanzgeräts weist die Felderzeugungseinheit ein Anschlusselement auf, welches dazu ausgebildet ist, eine Hochfrequenzspule des Hochfrequenzsystems und/oder eine Gradientenspule des Gradientensystems mit einer externen Stromquelle und/oder einem externen Kühlsystem zu verbinden. Das Anschlusselement der Felderzeugungseinheit ist als ein flexibles Verbindungselement ausgestaltet und dazu ausgebildet, relativ zu dem Hauptmagneten positioniert zu werden und innerhalb des von der Haltestruktur eingegrenzten Volumens verstaut zu werden.

Das flexible Verbindungselement kann beweglich ausgestaltet sein. Beispielsweise kann das flexible Verbindungselement einen beweglichen Schlauch und/oder ein bewegliches elektrisches Kabel umfassen. Das flexible Verbindungselement kann ferner einen elektrischen Anschluss und/oder einen Kühlanschluss umfassen. Der elektrische Anschluss und/oder der Kühlanschluss können an einem Anschlussende des flexiblen Verbindungselements angeordnet sein.

Vorzugsweise ist das flexible Verbindungselement derart beweglich ausgestaltet, dass das Anschlussende des flexiblen Verbindungselements bei Bedarf aus dem von dem durch den Hauptmagneten mit der Haltestruktur umschlossenen Volumen herausführbar ist. Gleichermaßen kann das flexible Verbindungselement dazu ausgebildet sein, in dem von der Haltestruktur eingegrenzten Volumen verstaut zu werden.

Durch das Bereitstellen eines flexiblen Verbindungselements, welches sich temporär in dem von der Haltestruktur eingegrenzten Volumen verstaut lässt, kann ein Überstehen von Komponenten der Felderzeugungseinheit über ein Ende der Haltestruktur des Hauptmagneten während des Transports des Magnetresonanzgeräts auf vorteilhafte Weise vermieden werden.

In einer Ausführungsform des erfindungsgemäßen Magnetresonanzgeräts ragt das Anschlusselement in ein von der Hochfrequenzspule umschlossenes Volumen und/oder einen Patientenaufnahmebereich des Magnetresonanzgeräts hinein.

Das Magnetresonanzgerät kann als ein geschlossener Scanner ausgestaltet sein, welcher einen zylindrisch geformten Hauptmagneten aufweist. Der zylindrisch geformte Hauptmagnet kann den Bildaufnahmebereich und/oder den Patientenaufnahmebereich entlang der Patientenzugangsrichtung und/oder entlang einer Hauptmagnetfeldrichtung umfänglich umschließen. Das Anschlusselement der Hochfrequenzspule kann beispielsweise in einer radialen Richtung des Hauptmagneten ausgerichtet sein. Insbesondere kann das Anschlusselement der Hochfrequenzspule in radialer Richtung des Hauptmagneten in den Patientenaufnahmebereich hineinragen.

Bei einem offenen Scanner mit zwei getrennten Magneten kann das Anschlusselement der Hochfrequenzspule in den Patientenaufnahmebereich, welcher sich zwischen den zwei Magneten erstreckt, hineinragen.

Vorzugsweise ist das Anschlusselement der Hochfrequenzspule derart in dem Patientenaufnahmebereich angeordnet, dass ein Blockieren des Patientenzugangs des Magnetresonanzgeräts vermieden wird. Beispielweise kann das Anschlusselement der Hochfrequenzspule an einem zweiten Ende des Magnetresonanzgeräts angeordnet sein, welches einem ersten Ende des Magnetresonanzgeräts mit dem Patientenzugangs gegenübersteht.

Eine oben beschriebene Anordnung des Anschlusselements der Hochfrequenzspule ermöglicht es, eine Dimension des Hauptmagneten vorteilhaft gegenüber konventionellen Magnetresonanzgeräten zu vergrößern oder zu maximieren.

In einer Ausführungsform des erfindungsgemäßen Magnetresonanzgeräts, weist eine Gradientenspule des Gradientensystems ein Anschlusselement auf. Das Anschlusselement der Gradientenspule ist dazu ausgebildet, das Gradientensystem mit einer externen Stromquelle und/oder einem externen Kühlsystem zu verbinden. Das Anschlusselement der Gradientenspule ist durch das von der Haltestruktur eingegrenzte Volumen außenumfänglich umschlossen.

Das Anschlusselement der Gradientenspule kann entsprechend einer Ausführungsform des Anschlusselements der Hochfrequenzspule ausgestaltet sein. Insbesondere kann das Anschlusselement der Gradientenspule einen elektrischen Anschluss und/oder einen Kühlanschluss umfassen.

Es ist vorstellbar, dass die Gradientenspule des Gradientensystems eine Aussparung aufweist, welche dazu ausgebildet ist, das Anschlusselement der Gradientenspule aufzunehmen.

Es ist weiterhin vorstellbar, dass das Anschlusselement der Gradientenspule zumindest teilweise in einer Vertiefung in der Wand der äußeren Vakuumkammer angeordnet und/oder verankert ist.

In einer Ausführungsform des erfindungsgemäßen Magnetresonanzgeräts weisen eine Hochfrequenzspule des Hochfrequenzsystems und/oder die Gradientenspule des Gradientensystems eine Aussparung auf, welche dazu ausgebildet ist, das Anschlusselement der Gradientenspule aufzunehmen.

Vorzugsweise weist die Hochfrequenzspule des Hochfrequenzsystems eine Aussparung auf, welche dazu ausgebildet ist, zumindest einen Teil des Anschlusselements der Gradientenspule aufzunehmen.

In einer Ausführungsform ist das Anschlusselement der Gradientenspule mechanisch mit dem Anschlusselement der Hochfrequenzspule integriert.

Es ist vorstellbar, dass die Gradientenspule des Gradientensystems und/oder die Hochfrequenzspule des Hochfrequenzsystems Aussparungen aufweisen, welche dazu ausgebildet sind, das Anschlusselement der Gradientenspule, das Anschlusselement der Hochfrequenzspule und/oder ein kombiniertes Anschlusselement, umfassend das Anschlusselement der Gradientenspule und das Anschlusselement der Hochfrequenzspule, aufzunehmen.

In einer Ausführungsform sind das Anschlusselement der Hochfrequenzspule und/oder das Anschlusselement der Gradientenspule in der Aussparung der Gradientenspule und/oder der Aussparung der Hochfrequenzspule mechanisch befestigt oder verankert.

In einer weiteren Ausführungsform des erfindungsgemäßen Magnetresonanzgeräts ist das Anschlusselement der Gradientenspule durch eine Aussparung in der Hochfrequenzspule hindurchgeführt und ragt in ein von der Hochfrequenzspule umschlossenes Volumen und/oder einen Patientenaufnahmebereich des Magnetresonanzgeräts hinein.

Das Anschlusselement der Gradientenspule kann analog zu dem Anschlusselement der Hochfrequenzspule in das von der Hochfrequenzspule umschlossene Volumen und/oder den Patientenaufnahmebereich des Magnetresonanzgeräts hineinragen. Vorzugsweise ist das Anschlusselement der Gradientenspule hierbei durch eine Ebene oder Schale der Hochfrequenzspule mit der Leiterstruktur hindurchgeführt.

Das erfindungsgemäße Anschlusselement der Gradientenspule teilt die Vorteile des erfindungsgemäßen Anschlusselements der Hochfrequenzspule.

In einer weiteren Ausführungsform umfasst das erfindungsgemäße Magnetresonanzsystem eine reversibel abnehmbare Gradienten-Anschlussplatte, welche dazu ausgebildet ist, eine Gradientenspule des Gradientensystems und/oder eine Hochfrequenzspule des Hochfrequenzsystems mit einer Stromquelle elektrisch und mechanisch zu verbinden.

Vorzugsweise ist die reversibel abnehmbare Gradienten-Anschlussplatte dazu ausgebildet, eine elektrische Anschlussleitung, welche die Gradientenspule mit der Stromquelle elektrisch verbindet, mechanisch zu befestigen. Die abnehmbare Gradienten-Anschlussplatte kann insbesondere dazu ausgebildet sein, die elektrische Anschlussleitung gegen Lorenzkräfte, welche während eines Betriebs des Magnetresonanzgeräts auftreten, zu stabilisieren. Es ist vorstellbar, dass die abnehmbare Gradienten-Anschlussplatte ein rigides Material aufweist, welches dazu ausgebildet ist, eine Verformung der abnehmbaren Gradienten-Anschlussplatte und/oder der elektrischen Anschlussleitung aufgrund von elektromagnetischen Kräften zu vermeiden.

Die Stromquelle kann eine externe Stromquelle darstellen. Vorzugsweise stellt die Stromquelle jedoch eine Gradientensteuereinheit und/oder eine Hochfrequenzeinheit des Magnetresonanzgeräts dar.

Die reversibel abnehmbare Gradienten-Anschlussplatte kann außerhalb des von der Haltestruktur eingegrenzten Volumens an dem Magnetresonanzgerät angeordnet sein. Insbesondere kann die abnehmbare Gradienten-Anschlussplatte an der Haltestruktur des Hauptmagneten reversibel befestigt oder montiert sein. Vorzugsweise ist die reversibel abnehmbare Gradienten-Anschlussplatte mittels einer reversiblen mechanischen Verbindung, insbesondere einer kraftschlüssigen und/oder einer formschlüssigen Verbindung, an der Haltestruktur des Hauptmagneten befestigt. Beispielsweise kann die abnehmbare Gradienten-Anschlussplatte mittels einer Schraubverbindung, einer Bolzenverbindung oder einer Klemmverbindung an der Haltestruktur des Hauptmagneten befestigt sein.

Durch das Vorsehen einer reversibel abnehmbaren Gradienten-Anschlussplatte lässt sich eine äußere Abmessung des Magnetresonanzgeräts temporär, z. B. für den Transport und/oder die Aufstellung des Magnetresonanzgeräts, reduzieren oder minimieren. Dadurch kann eine Abmessung des Hauptmagneten auf vorteilhafte Weise vergrößert oder maximiert werden.

In einer Ausführungsform umfasst das erfindungsgemäße Magnetresonanzgerät eine Halterung, welcher dazu ausgebildet ist, das Magnetresonanzgerät in einem vorbestimmten Abstand zu einer Bodenfläche zu halten.

Die Halterung kann eine beliebige mechanische Struktur umfassen, welche dazu ausgebildet ist, das Magnetresonanzgerät in einer vorbestimmten relativen Position zu der Bodenfläche zu befestigen. Die Halterung kann insbesondere dazu ausgebildet sein, den Hauptmagneten in einer vorbestimmten Orientierung zu der Bodenfläche zu halten. In einer bevorzugten Ausführungsform trägt die Halterung den Hauptmagneten. Die Halterung kann mechanisch mit der Haltestruktur des Hauptmagneten verbunden sein.

Die Halterung kann mittels einer geeigneten mechanischen Verbindung mit einer beliebigen Wand, insbesondere der Bodenfläche, einer Decke und/oder einer Seitenwand, eines Untersuchungsraums verbunden sein.

Erfindungsgemäß ist ein Teil der Halterung, welcher eine Abmessung der Haltestruktur in einer Raumrichtung überschreitet, reversibel abnehmbar ausgestaltet.

Der reversibel abnehmbare Teil der Halterung kann mittels einer beliebigen geeigneten mechanischen Verbindung mit der Halterung verbunden sein. Beispielsweise kann der reversibel abnehmbare Teil der Halterung mittels einer kraftschlüssigen und/oder einer formschlüssigen mechanischen Verbindung, wie z. B. einer Schraubverbindung, einer Klemmverbindung und/oder einer Bolzenverbindung, mit der Halterung verbunden sein.

Es ist vorstellbar, dass der reversibel abnehmbare Teil der Halterung bei anwendungsgemäßer Verbindung mit der Halterung in mindestens einer Raumrichtung, insbesondere in mindestens zwei Raumrichtungen, über das von der Haltestruktur eingegrenzte Volumen hinausragt. Beispielsweise kann die Halterung in einer Höhe und in einer Länge oder einer Breite über das von der Haltestruktur eingegrenzte Volumen hinausragen. Der reversibel abnehmbare Teil der Halterung kann derart ausgestaltet sein, dass die Halterung nach Abnehmen des reversibel abnehmbaren Teils der Halterung nur noch in einer Raumrichtung, z. B. einer Höhe, über das von der Haltestruktur umschlossene oder eingegrenzte Volumen hinausragt oder übersteht.

Der reversibel abnehmbare Teil der Halterung kann einen Abschnitt der Halterung, aber auch die gesamte Halterung umfassen. Beispielsweise kann die gesamte Halterung dazu ausgebildet sein, reversibel an der Haltestruktur befestigt zu werden.

In einer weiteren Ausführungsform umfasst das erfindungsgemäße Magnetresonanzgerät eine Halterung, welche dazu ausgebildet ist, das Magnetresonanzgerät in einem vorbestimmten Abstand zu einer Bodenfläche zu halten, wobei die Halterung relativ zu dem Hauptmagneten drehbar und/oder schwenkbar ausgestaltet ist.

Die Halterung kann entsprechend einer oben beschriebenen Ausführungsform ausgestaltet sein.

Es ist vorstellbar, dass die Halterung in einer Raumrichtung, welche bei dem Transport des Magnetresonanzgeräts limitierend ist, über das von der Haltestruktur eingegrenzte Volumen hinausragt oder übersteht. Beispielsweise kann die Halterung in einer Breite über das von der Haltestruktur umschlossene Volumen hinausragen, wobei die Breite des Magnetresonanzgeräts durch eine Breite eines standardisierten Zugangswegs (z. B. einer Tür oder eines Flurs) begrenzt ist.

Die Halterung kann drehbar mit der Haltestruktur verbunden sein, um eine temporäre Reduktion der Breite des Magnetresonanzgeräts und somit einen Transport durch den standardisierten Zugangsweg zu ermöglichen.

Es ist vorstellbar, dass die Halterung und/oder die Haltestruktur ein Gelenk oder einen Mechanismus aufweisen, welche dazu ausgebildet sind, die Halterung relativ zu dem Hauptmagneten mit der Haltestruktur zu rotieren und/oder zu schwenken. Das Gelenk oder der Mechanismus können z. B. als Gleitlager und/oder als ein Wälzlager ausgestaltet sein. Es ist vorstellbar, dass das Gelenk oder der Mechanismus ein Radiallager, ein Linearlager, ein Radiaxlager und/oder ein Axiallager umfassen. Insbesondere können das Gelenk oder der Mechanismus ein Scharnier und/oder ein Kugellager aufweisen. Eine erfindungsgemäße Halterung ermöglicht es, eine Abmessung des Magnetresonanzgeräts temporär, z. B. für den Transport und/oder die Aufstellung des Magnetresonanzgeräts, zu reduzieren. Dadurch lässt sich eine Abmessung des Hauptmagneten vorteilhaft vergrößern oder maximieren, da die Halterung mit der Abmessung des Hauptmagneten skalieren kann, aber sich zum Zwecke des Transports in wenigstens einer Raumrichtung temporär verkleinern lässt kann.

In einer Ausführungsform umfasst das erfindungsgemäße Magnetresonanzgerät eine Außenhülle mit einem reversibel abnehmbaren Abschnitt.

Die Außenhülle des Magnetresonanzgeräts kann ein beliebiges Gehäuse oder Cover darstellen, welches technische Komponenten des Magnetresonanzgeräts verkleidet und/oder vor einer mechanischen Einwirkung von außen schützt. Die Außenhülle kann ebenso dazu ausgebildet sein, eine Person vor einem direkten Kontakt mit den technischen Komponenten des Magnetresonanzgeräts zu schützen.

Vorzugsweise umfasst die Außenhülle mindestens einen reversibel abnehmbaren Abschnitt. Es ist vorstellbar, dass der reversibel abnehmbare Abschnitt dazu ausgebildet ist, reversibel mechanisch mit der Außenhülle und/oder der Haltestruktur des Hauptmagneten verbunden zu werden.

Gemäß der Erfindung ist eine Benutzerschnittstelle, welche von dem abnehmbaren Abschnitt getragen ist, mittels einer elektrischen Schnittstelle mit einer Steuereinheit des Magnetresonanzgeräts verbunden, wobei eine elektrische Anschlussleitung, welche die Benutzerschnittstelle mit der elektrischen Schnittstelle verbindet, so ausgestaltet ist, dass ein reversibles abnehmen des abnehmbaren Abschnitts mit der Benutzerschnittstelle von dem Magnetresonanzgerät ermöglicht wird.

Die elektrische Schnittstelle kann dazu ausgebildet sein, eine direkte mechanische Verbindung zwischen der Benutzerschnittstelle und einer Steuereinheit des Magnetresonanzgeräts mittels elektrischer Leitungen zu vermeiden. Vorzugsweise ist eine Länge einer elektrischen Leitung, welche die Benutzerschnittstelle mit der elektrischen Schnittstelle verbindet, ausreichend, um ein Abnehmen des reversibel abnehmbaren Abschnitts von dem Magnetresonanzgerät zu ermöglichen, ohne die elektrische Leitung, die Benutzerschnittstelle und/oder die elektrische Schnittstelle zu beschädigen.

Die Benutzerschnittstelle kann ein Bedienpanel oder ein HMI (*"human-machine-interface"*) Panel des Magnetresonanzgeräts darstellen. Es ist vorstellbar, dass die Benutzerschnittstelle mechanisch mit dem reversibel abnehmbaren Abschnitt der Außenhülle verbunden ist.

Durch das Vorsehen eines reversibel abnehmbaren Abschnitts der Außenhülle lässt sich eine äußere Abmessung des Magnetresonanzgeräts temporär, z. B. für den Transport und/oder die Aufstellung des Magnetresonanzgeräts, reduzieren oder minimieren. Dadurch kann eine Abmessung des Hauptmagneten auf vorteilhafte Weise vergrößert oder maximiert werden.

In einer weiteren Ausführungsform umfasst das erfindungsgemäße Magnetresonanzgerät eine Außenhülle, wobei ein Abschnitt der Außenhülle den Hauptmagneten entlang eines Abschnitts einer Patientenzugangsrichtung umschließt und wobei eine Abmessung des Abschnitts der Außenhülle entlang der Patientenzugangsrichtung eine Abmessung der Haltestruktur entlang der Patientenzugangsrichtung unterschreitet.

Der Abschnitt der Außenhülle kann den Hauptmagneten, alle Magneten des Hauptmagneten, die Felderzeugungseinheit und/oder die Haltestruktur des Magnetresonanzgeräts entlang des Abschnitts der Patientenzugangsrichtung umschließen.

Der Abschnitt der Außenhülle kann einteilig ausgestaltet oder aus einer Mehrzahl von Teilen zusammengesetzt sein.

Es ist vorstellbar, dass das Magnetresonanzgerät als ein geschlossener Scanner ausgestaltet ist. Eine Länge des Abschnitts der Außenhülle kann eine Länge der Haltestruktur in axialer Richtung, insbesondere entlang einer Zylinderachse, des Magnetresonanzgeräts unterschreiten.

Vorzugsweise weist die Außenhülle wenigstens einen Endabschnitt auf, welcher dazu ausgebildet ist, mit einem distalen Abschnitt oder einem axialen Ende der Haltestruktur reversibel mechanisch verbunden zu werden. Der distale Abschnitt oder das axiale Ende der Haltestruktur kann insbesondere eine Grundfläche oder eine Deckfläche eines zylindrischen geformten Körpers der Haltestruktur darstellen. Es ist vorstellbar, dass der wenigstens eine Endabschnitt der Außenhülle den distalen Abschnitt oder das axiale Ende der Haltestruktur derart umgreift, dass der wenigstens eine Endabschnitt auch einen Abschnitt des Hauptmagneten entlang der Patientenzugangsrichtung oder der Zylinderachse des Hauptmagneten umschließt.

In einer bevorzugten Ausführungsform schließt der Abschnitt der Außenhülle mit dem wenigstens einen Endabschnitt ab, sodass der Hauptmagnet mit der Haltestruktur vollständig von der Außenhülle umschlossen sind.

Die erfindungsgemäße Außenhülle ermöglicht ein reversibles Entfernen von Abschnitten der Außenhülle, insbesondere wenigstens eines Endabschnitts der Außenhülle, welche in einem montierten Zustand am Magnetresonanzgerät ein Maß eines standardisierten Zugangswegs überschreiten würden. Durch das Vorsehen eines Abschnitts der Außenhülle, welcher eine Dimension des Hauptmagneten entlang einer Haupterstreckungsrichtung unterschreitet, lässt sich ein Überstehen über eine Querschnittsfläche des Magnetresonanzgeräts, welche mit einer Abmessung des standardisierten Zugangswegs abgestimmt ist, auf vorteilhafte Weise vermeiden.

Weitere Vorteile und Einzelheiten ergeben sich aus der nachfolgenden Beschreibung von Ausführungsbeispielen in Verbindung mit den Zeichnungen. Es zeigen in Prinzipdarstellung:
- Fig. 1: ein konventionelles Magnetresonanzgerät,
- Fig. 2: eine Ausführungsform eines erfindungsgemäßen Magnetresonanzgeräts,
- Fig. 3: eine Ausführungsform eines erfindungsgemäßen Magnetresonanzgeräts,
- Fig. 4: eine Ausführungsform eines erfindungsgemäßen Magnetresonanzgeräts,
- Fig. 5: eine Ausführungsform eines erfindungsgemäßen Magnetresonanzgeräts,
- Fig. 6: eine Ausführungsform eines erfindungsgemäßen Magnetresonanzgeräts,
- Fig. 7: eine Ausführungsform eines erfindungsgemäßen Magnetresonanzgeräts,
- Fig. 8: eine Ausführungsform eines erfindungsgemäßen Magnetresonanzgeräts,
- Fig. 9: eine Ausführungsform eines erfindungsgemäßen Magnetresonanzgeräts,
- Fig. 10: eine Ausführungsform eines erfindungsgemäßen Magnetresonanzgeräts,
- Fig. 11: eine Ausführungsform eines erfindungsgemäßen Magnetresonanzgeräts.

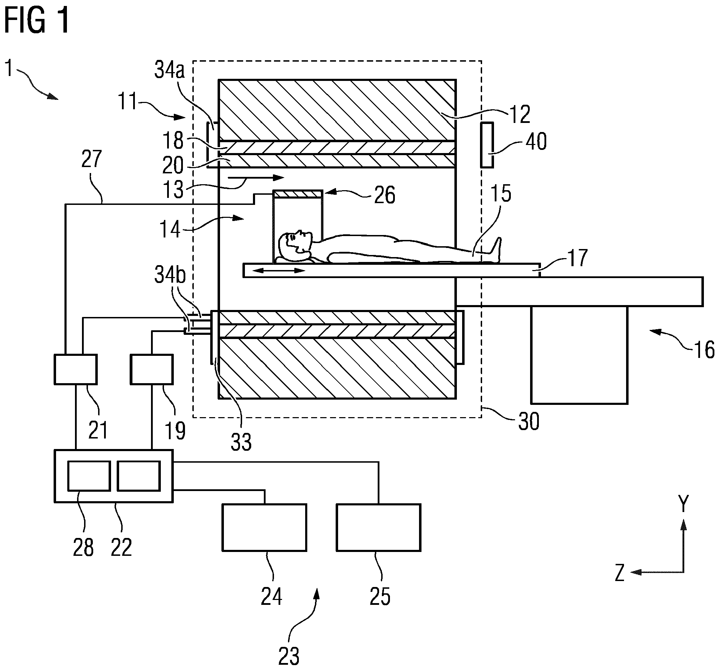

In Fig. 1 ist ein konventionelles Magnetresonanzgerät 1 dargestellt. Das Magnetresonanzgerät 1 umfasst eine Felderzeugungseinheit 11, welche einen Hauptmagneten 12 mit einem oder mehreren Permanentmagneten, Elektromagneten oder supraleitenden Magneten zur Erzeugung eines starken und insbesondere homogenen Hauptmagnetfelds 13 (B0-Magnetfeld) aufweist. Zudem umfasst das Magnetresonanzgerät 1 einen Patientenaufnahmebereich 14 zu einer Aufnahme eines Patienten 15. Der Patientenaufnahmebereich 14 ist im gezeigten Ausführungsbeispiel zylinderförmig ausgebildet und in einer Umfangsrichtung von dem Hauptmagneten 11 umschlossen. Grundsätzlich sind jedoch auch von diesem Beispiel abweichende Ausbildungen des Patientenaufnahmebereichs 14 vorstellbar. Der Patientenaufnahmebereich 14 kann im Wesentlichen mit einem Bildaufnahmebereich des Magnetresonanzgeräts 1 übereinstimmen.

In dem in der Fig. 1 gezeigten Beispiel ist das Untersuchungsobjekt ein Patient 15. Der Patient 15 kann mittels einer Patientenlagerungsvorrichtung 16 des Magnetresonanzgeräts 1 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf.

Die Felderzeugungseinheit 11 weist weiterhin ein Gradientensystem mit wenigstens einer Gradientenspule 18 zum Erzeugen von magnetischen Gradientenfeldern auf, welche für eine Ortskodierung während einer Magnetresonanzmessung verwendet wird. Die Gradientenspule 18 wird mittels einer Gradientensteuereinheit 19 des Magnetresonanzgeräts 1 angesteuert. Es ist denkbar, dass das Gradientensystem mehrere Gradientenspulen 18 zur Erzeugung magnetischer Gradientenfelder in unterschiedlichen, vorzugsweise orthogonal zueinander ausgerichteten, Raumrichtungen umfasst.

Die Felderzeugungseinheit 11 umfasst außerdem ein Hochfrequenzsystem mit einer Hochfrequenzspule, welche im vorliegenden Ausführungsbeispiel als fest in das Magnetresonanzgerät 1 integrierte Körperspule 20 ausgebildet ist. Die Körperspule 20 ist zu einer Anregung von Kernspins ausgelegt, die sich in dem von dem Hauptmagneten 12 erzeugten Hauptmagnetfeld 13 befinden. Die Körperspule 20 wird von einer Hochfrequenzeinheit 21 des Magnetresonanzgeräts 1 angesteuert und strahlt hochfrequente Anregungspulse in den Bildaufnahmebereich, der im Wesentlichen von dem Patientenaufnahmebereich 14 des Magnetresonanzgeräts 1 gebildet ist, ein. Die Körperspule 20 ist weiterhin zum Empfang von Magnetresonanzsignalen ausgebildet und kann eine Empfangseinheit oder einen Teil einer Empfangseinheit des Magnetresonanzgeräts 1 darstellen.

Zu einer Steuerung des Magnetresonanzgeräts 1, insbesondere der Gradientensteuereinheit 19 und der Hochfrequenzeinheit 21, weist das Magnetresonanzgerät 1 eine Steuereinheit 22 auf. Die Steuereinheit 22 ist dazu ausgebildet, eine Durchführung einer Bildgebungssequenz, wie z. B. einer GRE (gradient echo) Sequenz, einer TSE (turbo spin echo) Sequenz oder einer UTE (ultra-short echo time) Sequenz, zu steuern. Zudem umfasst die Steuereinheit 22 eine Recheneinheit 28 zu einer Auswertung von Magnetresonanzsignalen, die während einer Magnetresonanzmessung mit einer Bildgebungssequenz erfasst werden.

Das Magnetresonanzgerät 1 kann eine Benutzerschnittstelle 23 umfassen, welche eine Signalverbindung mit der Steuereinheit 22 aufweist. Steuerinformationen, wie z. B. Bildgebungsparameter der Magnetresonanzmessung, können auf einer Anzeigeeinheit 24, beispielsweise auf zumindest einem Monitor, der Benutzerschnittstelle 23 angezeigt werden. Die Anzeigeeinheit 24 kann insbesondere dazu ausgelegt sein, eine grafische Benutzeroberfläche mit der Darstellung einer relevanten Körperregion des Patienten 15 bereitzustellen. Weiterhin weist die Benutzerschnittstelle 23 eine Eingabeeinheit 25 auf, mittels welcher Parameter einer Magnetresonanzmessung von einem Nutzer eingegeben oder verändert werden können.

Das Magnetresonanzgerät 1 kann weitere Komponenten, wie z. B. eine Lokalspule 26, aufweisen. Die Lokalspule 26 kann in einer anwendungsgemäßen Position an einer diagnostisch oder therapeutisch relevanten Körperregion des Patienten 15 positioniert sein. Die Lokalspule 26 weist vorzugsweise eine Mehrzahl von Antennenelementen auf, welche dazu ausgebildet sind, Magnetresonanzsignale der relevanten Körperregion des Patienten 15 zu erfassen und an die Recheneinheit 28 und/oder die Steuereinheit 22 zu übermitteln. Die Lokalspule kann hierfür mittels einer elektrischen Anschlussleitung 27 oder einer anderen Signalverbindung mit der Hochfrequenzeinheit 21 und der Steuereinheit 22 verbunden sein. Analog zu der Körperspule 20 kann auch die Lokalspule 26 zu einem Anregen von Kernspins in der Kieferregion 31 des Patienten 15 ausgebildet sein. Die Lokalspule 26 kann hierfür von der Hochfrequenzeinheit 21 angesteuert werden.

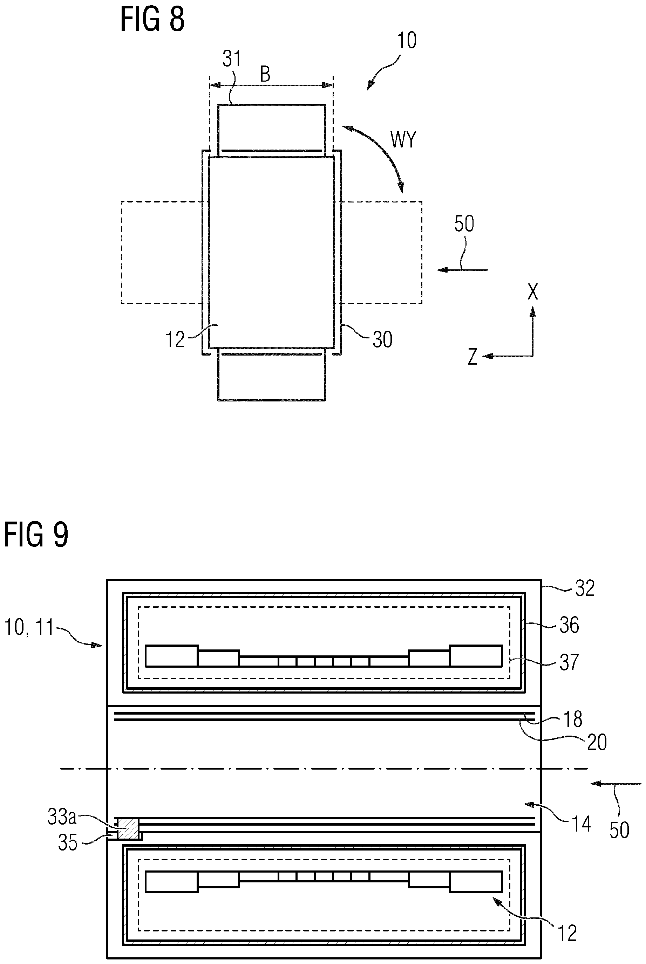

Typischerweise weisen konventionelle Magnetresonanzgeräte 1 Komponenten auf, welche über ein durch die Haltestruktur 32 eingegrenztes Volumen (siehe Fig. 9) hinausragen oder überstehen. Solche Teile können insbesondere Abschnitte oder Komponenten der Felderzeugungseinheit 11, wie z. B.
- elektrische Anschlüsse 34b der Gradientenspule 18 und/oder der Körperspule 20,
- Trägerstrukturen 33 für die Körperspule 20,
- Kühlanschlüsse 34a für die Gradientenspule 20 und/oder die Körperspule 18,
- Teile der Außenhülle 30 und/oder
- Teile einer Benutzerschnittstelle, wie z. B. ein HMI-Panel 40 an der Außenhülle 30,
sein.

Konventionelle Ganzkörper-Magnetresonanzgeräten 1 werden üblicherweise mittels dedizierter Hebeeinrichtungen über vorbestimmte Transportwege in klinische Einrichtungen transportiert, weshalb eine vergrößerte Außenabmessung durch überstehende Teile meist unproblematisch ist.

Bei dedizierten Scannern, welche aufgrund kleinerer Außenabmessungen auch über standardisierte Zugangswege in kleinere klinische Einrichtungen und Praxen transportiert werden sollen, können solche überstehenden Teile jedoch ein großes Problem darstellen.

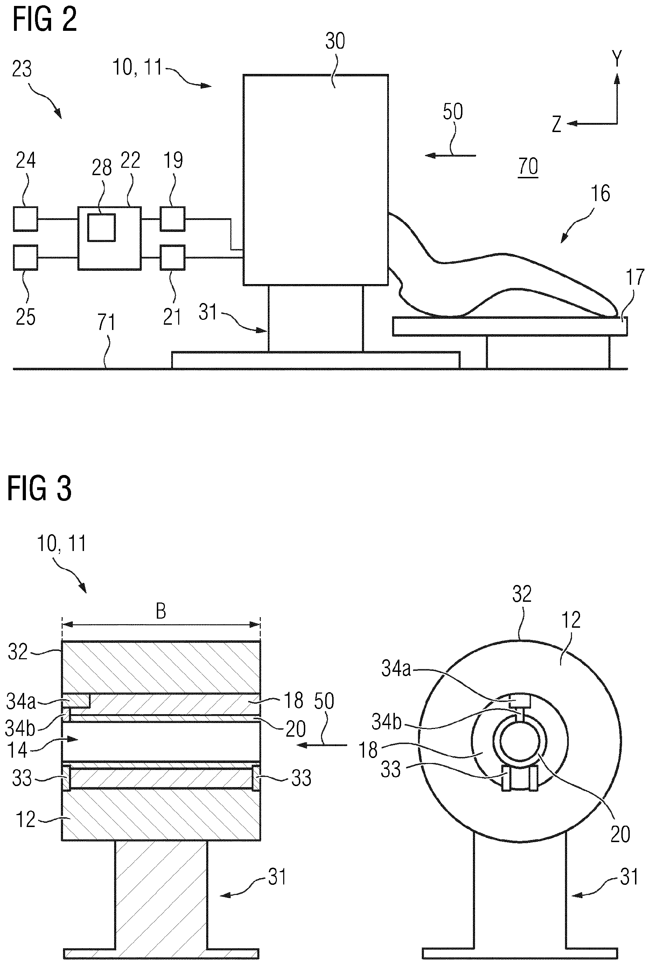

Fig. 2 zeigt eine Ausführungsform eines erfindungsgemäßen Magnetresonanzgeräts 10. Grundsätzlich stimmen die Funktionen und Komponenten des in Fig. 2 exemplarisch dargestellten Magnetresonanzgeräts 10 mit den oben beschriebenen Funktionen und Komponenten eines konventionellen Magnetresonanzgeräts 1 (siehe Fig. 1) überein.

Beispielsweise kann das Magnetresonanzgerät 10 dazu ausgebildet sein, eine Magnetresonanzuntersuchung einer Kieferregion und/oder einer Augenregion eines Patienten 15 durchzuführen. Das erfindungsgemäße Magnetresonanzgerät 10 kann ebenso dazu ausgebildet sein, eine kardiale Bildgebung, eine Mammographie-Bildgebung, eine neurologische Bildgebung, eine urologische Bildgebung, eine orthopädische Bildgebung, eine Prostata-Bildgebung oder eine Bildgebung anderer Körperregionen des Patienten 15 durchzuführen.

In dem in Fig. 2 gezeigten Beispiel wird das Magnetresonanzgerät 10 von einer Halterung 31 getragen und in einem vorbestimmten Abstand zu einer Bodenfläche 71 eines Untersuchungsraums 70 gehalten. Es ist vorstellbar, dass die Halterung 31 eine Positionierungseinheit (nicht gezeigt) aufweist, welche dazu ausgebildet ist, die Felderzeugungseinheit 11 des Magnetresonanzgeräts 10 relativ zu einer diagnostisch relevanten Körperregion des Patienten 15 zu positionieren und/oder auszurichten. Zum Beispiel kann die Positionierungseinheit ein Drehgelenk umfassen, welches dazu ausgebildet ist, die Felderzeugungseinheit 11 entlang einer Drehrichtung zu drehen. Eine räumliche Position der Felderzeugungseinheit 11 entlang einer Y-Richtung und/oder einer Z-Richtung kann über ein geeignetes Teleskopsystem und/oder Schienensystem, welches mechanisch mit der Halterung 31 gekoppelt ist, eingestellt werden.

Es ist ebenso vorstellbar, dass das Magnetresonanzgerät 10 eine in Fig. 2 dargestellte Patientenlagerungsvorrichtung 16 und/oder einen Patiententisch 17 umfasst, welcher dazu ausgebildet ist, eine diagnostisch relevante Körperregion des Patienten 15 in dem Bildaufnahmebereich zu positionieren.

Abweichend von der in Fig. 2 gezeigten Ausführungsform kann die Halterung 31 auch dazu ausgebildet sein, das Magnetresonanzgerät 10 bzw. die Felderzeugungseinheit 11 an einer Wand und/oder einer Decke eines Untersuchungsraums 70 zu befestigen.

Das dargestellte Magnetresonanzgerät 10 kann selbstverständlich weitere Komponenten enthalten, welche Magnetresonanzgeräte üblicherweise umfassen. Die allgemeine Funktionsweise eines Magnetresonanzgeräts ist dem Fachmann wohlbekannt. Auf eine detaillierte Beschreibung weiterer Bestandteile oder einer Messdatenerfassung einer Magnetresonanzuntersuchung wird daher verzichtet.

Es ist ebenso vorstellbar, dass das Magnetresonanzgerät 10 statt des zylinderförmigen Aufbaus einen C-förmigen, einen dreieckigen oder einen asymmetrischen Aufbau der Felderzeugungseinheit 11 aufweist. Das Magnetresonanzgerät 10 kann insbesondere ein dedizierter Scanner sein, welcher dazu ausgebildet ist, eine Magnetresonanzbildgebung einer Kieferregion und/oder Kopfregion eines stehenden oder sitzenden Patienten 15 durchzuführen. Die folgenden Figuren 3 bis 7 zeigen weitere Aspekte des erfindungsgemäßen Magnetresonanzgeräts 10 im Detail.

Fig. 3 zeigt eine Ausführungsform des erfindungsgemäßen Magnetresonanzgeräts 10 im Querschnitt. In dem vorliegenden Beispiel weist die Körperspule 20 mehrere Trägerstrukturen 33 auf, welche durch Aussparungen in der Gradientenspule 18 hindurchragen und die Körperspule 20 mechanisch mit der Haltestruktur 32 des Hauptmagneten 12 (vgl. Figs. 8 bis 10) verbinden. Ein Überstehen oder Hinausragen der Trägerstrukturen 33 über ein axiales Ende des Hauptmagneten 12 und der Haltestruktur 32 wird somit vermieden.

In Figs. 8 bis 10 sind schematisch mehrere Möglichkeiten gezeigt, wie die Körperspule 20 mittels einer Trägerstruktur 33 an der Gradientenspule 18 und/oder der Haltestruktur 32 befestigt sein kann.

In dem in Fig. 3 gezeigten Beispiel ist der Kühlanschluss 34a für die Gradientenspule 18, und optional für die Körperspule 20, in einer Aussparung der Gradientenspule 18 aufgenommen. Vorzugsweise sind Leiterstrukturen der Gradientenspule 18 um die Aussparung in einem Material der Gradientenspule 18 herumgelegt (nicht gezeigt).

Gleichermaßen ist ein elektrischer Anschluss 34b, welcher die Gradientenspule 18 elektrisch mit der Gradientensteuereinheit 19 verbindet (siehe Fig. 2), in einer entsprechenden Aussparung in der Gradientenspule 18 und der Körperspule 20 aufgenommen.

Der elektrische Anschluss 34b, aber auch der Kühlanschluss 34a, können Anschlusselemente 34 darstellen, welche in einer entsprechenden Aussparung in der Gradientenspule 18 und/oder der Körperspule 20 aufgenommen oder untergebracht sind. Die Anschlusselemente 34 sind vorzugsweise dazu ausgebildet, angeschlossene elektrische Leitungen, aber auch Kühlanschlüsse, mechanisch zu befestigen und/oder gegen Lorenzkräfte zu stabilisieren.

Durch die Aufnahme der elektrischen Anschlüsse 34b, der Trägerstrukturen 33 und der Kühlanschlüsse 34a in Aussparungen der Gradientenspule 18 und/oder der Körperspule 20 lässt sich eine Breite B des erfindungsgemäßen Magnetresonanzgeräts 10 auf ein standardisiertes Maß, z. B. eine Breite von unter 80 cm, reduzieren. Gleichzeitig kann der Hauptmagneten 12 auf die zur Verfügung stehende Breite B hin dimensioniert werden und somit eine höhere Magnetfeldstärke und/oder eine verbesserte Homogenität des Hauptmagnetfelds bereitstellen.

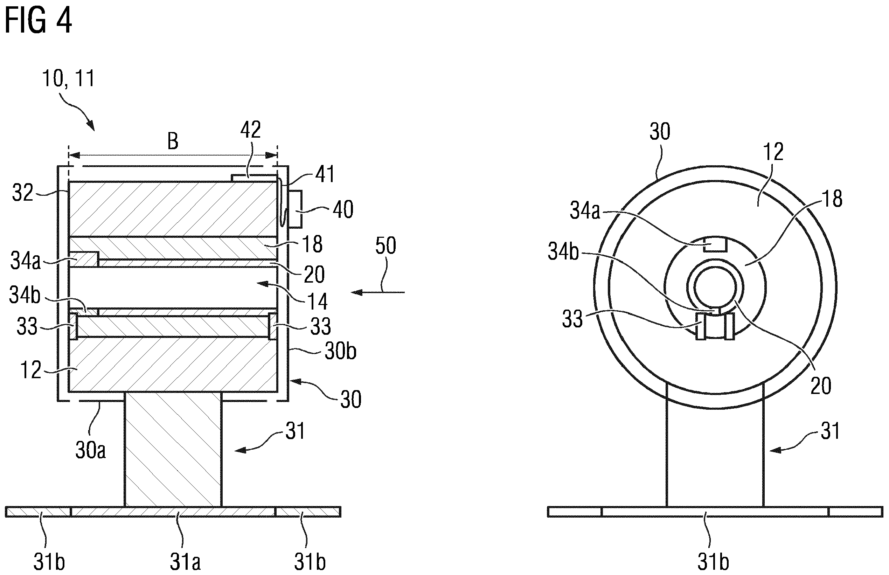

In der in Fig. 4 gezeigten Ausführungsform sind die elektrischen Anschlüsse 34b der Gradientenspule 18 und der Körperspule 20 in einer Aussparung in der Körperspule 20 aufgenommen. Es ist aber ebenso vorstellbar, dass die elektrischen Anschlüsse 34b für die Gradientenspule 18 und die Körperspule 20 in einer Aussparung in der Gradientenspule 18 und/oder einer Aussparung der Körperspule 20 aufgenommen oder angeordnet sind.

Der Kühlanschluss 34a des Gradientensystems 18 ist vorliegend in einer Aussparung in der Körperspule 20 angeordnet. Der Kühlanschluss 34a kann ebenso in einer Aussparung der Gradientenspule 18 und/oder der Körperspule 20 angeordnet sein.

Die in Fig. 4 gezeigte Ausführungsform des erfindungsgemäßen Magnetresonanzgeräts 10 weist eine Außenhülle 30 mit einem reversibel abnehmbaren Abschnitt 30b auf. Der reversibel abnehmbare Abschnitt 30b trägt eine Benutzerschnittstelle 40, welche beispielsweise als ein HMI-Panel oder ein Tablet mit einer Dockingstation ausgestaltet ist. Vorzugsweise ist die Benutzerschnittstelle 40 elektrisch mit der Steuereinheit 22 des Magnetresonanzgeräts 10 verbunden und ermöglicht einem Nutzer eine Steuerung von Funktionen des Magnetresonanzgeräts 10.

Die Benutzerschnittstelle 40 ist mittels einer elektrischen Anschlussleitung 41 mit einer elektrischen Schnittstelle 42 verbunden, welche wiederum mit der Steuereinheit 22 des Magnetresonanzgeräts 10 verbunden ist. Die elektrische Anschlussleitung 41 ist so ausgestaltet, dass ein Abnehmen des abnehmbaren Abschnitts 30b der Außenhülle 30 mit der Benutzerschnittstelle 40 von dem Magnetresonanzgerät 10 ermöglicht wird. Insbesondere weist die elektrische Anschlussleitung 41 eine Länge auf, welche ein Abnehmen des abnehmbaren Abschnitts 30b ermöglicht, ohne die elektrische Anschlussleitung 41 von der elektrischen Schnittstelle 42 und der Benutzerschnittstelle 40 mechanisch zu trennen. Vorzugsweise ist die elektrische Schnittstelle 42 derart angeordnet, dass ein Hinausragen über eine Breite B des Hauptmagneten und der Haltestruktur 32 des Hauptmagneten 12 vermieden wird. Die elektrische Schnittstelle 42 kann hierfür an einer Außenseite, insbesondere einer radialen Außenseite der Haltestruktur 32 angeordnet sein.

Weiterhin weist die Außenhülle 30 einen Abschnitt 30a auf, welcher den Hauptmagneten 12 entlang eines Abschnitts der Patientenzugangsrichtung 50 umschließt. In dem in Fig. 4 gezeigten Beispiel unterschreitet eine Abmessung des Abschnitts 30a der Außenhülle entlang der Patientenzugangsrichtung 50 eine Abmessung des Hauptmagneten 12 und der Haltestruktur 32 entlang der Patientenzugangsrichtung 50. Es ist vorstellbar, dass der Abschnitt 30a auch während des Transports an dem Magnetresonanzgerät 10 befestigt sein kann, da die Breite B des Magnetresonanzgeräts 10 nicht durch den Abschnitt 30a der Außenhülle 30 vergrößert wird.

In dem in Fig. 4 gezeigten Beispiel weist das Magnetresonanzgerät 10 weiterhin eine Halterung 31 mit abnehmbaren Teilen 31b auf. Die abnehmbaren Teile 31b überschreiten die Breite B der Haltestruktur 32 entlang der Patientenzugangsrichtung 50, wenn sie anwendungsgemäß an der Halterung 31 montiert sind. Vorzugsweise sind die abnehmbaren Teile 31b der Halterung 31 reversibel abnehmbar ausgestaltet. Die abnehmbaren Teile 31b können mittels einer beliebigen geeigneten mechanischen Verbindung reversibel mit der Halterung 31 verbunden sein. Durch das Abnehmen der Teile 31b von der Halterung 31 kann ein Überschreiten der Breite B des Magnetresonanzgeräts 10 bei einem Transport vermieden werden.

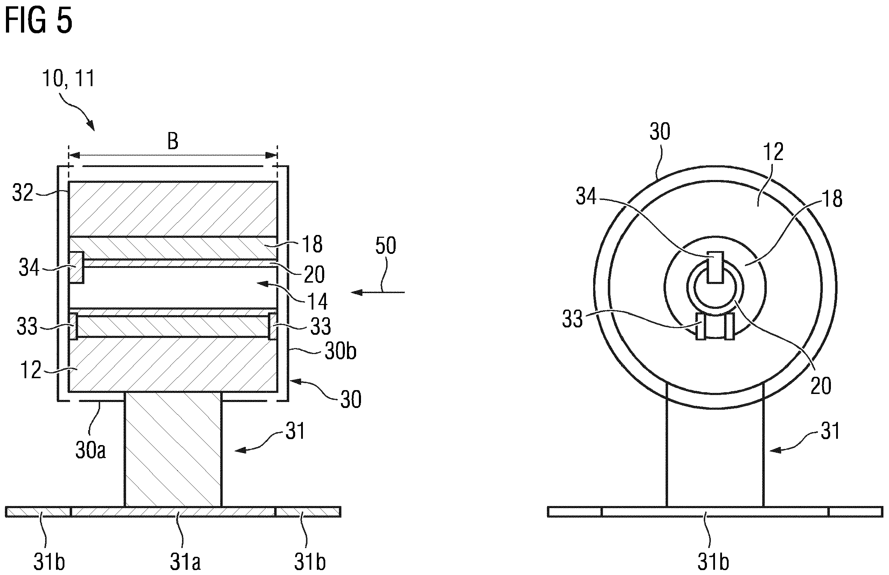

Fig. 5 zeigt eine weitere Ausführungsform des erfindungsgemä-ßen Magnetresonanzgeräts 10. In dem dargestellten Beispiel ragen ein oder mehrere Anschlusselemente 34 der Gradientenspule 18 und/oder der Körperspule 20 in ein von der Körperspule 20 umschlossenes Volumen und/oder den Patientenaufnahmebereich 14 des Magnetresonanzgeräts 10 hinein. Es ist vorstellbar, dass Abschnitte der Anschlusselemente 34 hierfür durch die Körperspule 20, und optional auch die Gradientenspule 18, hindurchgeführt sind. Die Körperspule 20, aber auch die Gradientenspule 18, können eine Aussparung umfassen, welche dazu ausgebildet sind, die Anschlusselemente 34 aufzunehmen.

Die Anschlusselemente 34 können einen oder mehrere elektrische Anschlüsse und/oder Kühlanschlüsse der Gradientenspule 18 und/oder der Körperspule 20 umfassen. Es ist ebenso vorstellbar, dass elektrische Anschlüsse und Kühlanschlüsse als getrennte Anschlusselemente 34 vorliegen und an unterschiedlichen Positionen entlang einer Innenoberfläche des Patientenaufnahmebereichs 14 in den Patientenaufnahmebereich 14 hineinragen.

Vorzugsweise sind die Anschlusselemente 34 an einem Ende, insbesondere einem axialen Ende, des Hauptmagneten 12 oder der Haltestruktur 32 angeordnet, um eine Kollision mit einem Patienten 15 während einer Magnetresonanzuntersuchung mit dem Magnetresonanzgerät 10 zu vermeiden.

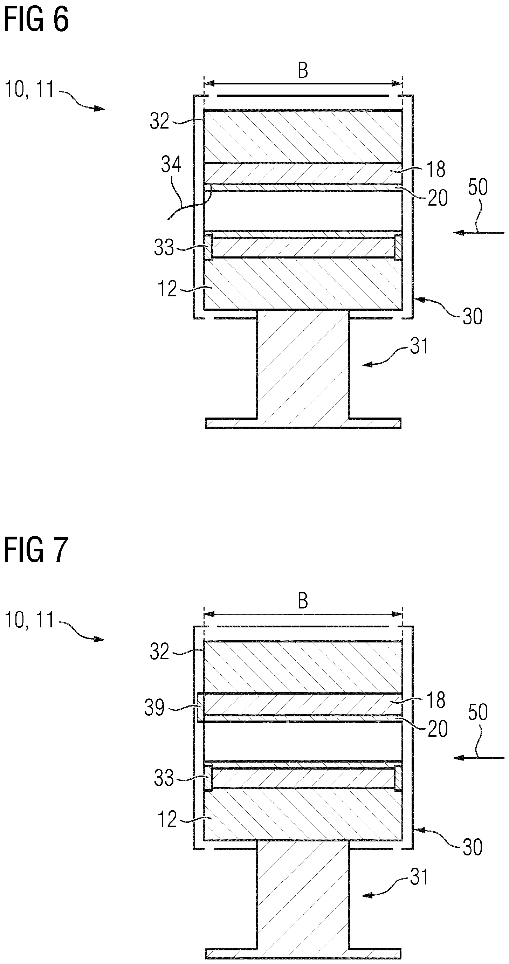

Fig. 6 zeigt eine Ausführungsform des erfindungsgemäßen Magnetresonanzgeräts 10, bei welcher ein Anschlusselement 34 der Gradientenspule 18 als flexibles Verbindungselement ausgestaltet ist. Das flexible Verbindungselement kann dazu ausgebildet sein, temporär innerhalb des durch die Haltestruktur 32 eingegrenzten Volumens verstaut zu werden. Vorzugsweise umfasst das flexible Verbindungselement 34 die elektrischen Anschlüsse, welche für eine elektrische Verbindung der Gradientenspule 18 mit der Gradientensteuereinheit 19 (siehe

Fig. 2) erforderlich sind. Das flexible Verbindungselement kann aber ebenso einen Kühlanschluss umfassen, welcher dazu ausgebildet ist, die Gradientenspule 18 an einen externen Kühlkreislauf anzuschließen.

In dem vorliegenden Beispiel ist das flexible Verbindungselement durch eine Aussparung in der Körperspule 20 hindurchgeführt.

Es ist vorstellbar, dass auch die Körperspule 20 ein flexibles Verbindungselement (nicht gezeigt) aufweist, welches dazu ausgebildet ist, die Körperspule 20 elektrisch mit der Hochfrequenzeinheit 21 zu verbinden. Weiterhin kann auch das flexible Verbindungselement der Körperspule 20 einen Kühlanschluss umfassen, welcher dazu ausgebildet ist, die Körperspule 20 an einen externen Kühlkreislauf anzuschließen.

Fig. 7 zeigt eine Ausführungsform des erfindungsgemäßen Magnetresonanzgeräts 10 mit einer reversibel abnehmbaren Anschlussplatte 39. Vorzugsweise ist die reversibel abnehmbare Anschlussplatte 39 dazu ausgebildet, zum Zwecke des Transports reversibel von der Haltestruktur 32 und/oder der Felderzeugungseinheit 11 demontiert zu werden, um die Haltestruktur 32 auf die Breite B zu beschränken. Die reversibel abnehmbare Anschlussplatte 39 kann elektrische Anschlüsse für die Gradientenspule 18 und/oder die Körperspule 20 umfassen.

ES ist jedoch ebenso vorstellbar, dass die reversibel abnehmbare Anschlussplatte 39 einen Kühlanschluss für die Gradientenspule 18 und/oder die Körperspule 20 umfasst.

Fig. 8 zeigt eine Ausführungsform des erfindungsgemäßen Magnetresonanzgeräts 10 mit einer drehbaren Halterung 31. Die Halterung 31 weist vorliegend ein Lager oder ein Gelenk auf, welches dazu ausgebildet ist, ein Rotieren der Halterung 31 entlang der Drehrichtung WY zu ermöglichen. Somit lassen sich über eine Breite B der Haltestruktur 32 überstehende Teile der Halterung 31 temporär rotieren oder verschwenken. Beispielsweise kann die Halterung 31 entlang der Z-Richtung eine größere Abmessung als in X-Richtung aufweisen. Mittels des Lagers oder Gelenks kann ein Teil der Halterung 31 mit der längeren Abmessung temporär entlang der X-Richtung ausgerichtet werden, sodass ein kürzerer Teil der Halterung 31 entlang der Z-Richtung ausgerichtet ist und einen Transport des Magnetresonanzgeräts durch standardisierte Zugangswege ermöglicht.

Fig. 9 zeigt eine Ausführungsform des erfindungsgemäßen Magnetresonanzgeräts 10, bei welcher die Trägerstruktur 33a der Körperspule 20 in einer Vertiefung 35 in der Wand der äußeren Vakuumkammer aufgenommen und befestigt ist. Die Vertiefung 35 kann eine Aussparung in einem Material der Wand der äußeren Vakuumkammer darstellen. Es ist jedoch ebenso vorstellbar, dass die Vertiefung 35 als eine Senke oder Mulde ausgestaltet ist, welche z. B. durch einen Tiefziehprozess erhalten werden kann. Die Wand der äußeren Vakuumkammer kann an der Vertiefung 35 in Richtung des Hauptmagneten 12 in ein von der äußeren Vakuumkammer umschlossenes Volumen hineinragen.

Die äußere Vakuumkammer stellt ein Teil der Haltestruktur 32 des Hauptmagneten 12 dar. Das von der Haltestruktur 32 eingegrenzte Volumen umfasst in dem gezeigten Beispiel den von der Haltestruktur 32 umschlossenen Patientenaufnahmebereich 14, sowie den von der Haltestruktur 32 eingeschlossenen Vakuumbereich, in welchem der Hauptmagnet 12 und eine thermische Abschirmung 36 angeordnet sind. Der Hauptmagnet 12 ist vorliegend von der thermischen Abschirmung 36 und einem optionalen Kryobehälter 37 (bei einem "nassen" Magnetresonanzgerät) außenumfänglich umschlossen.

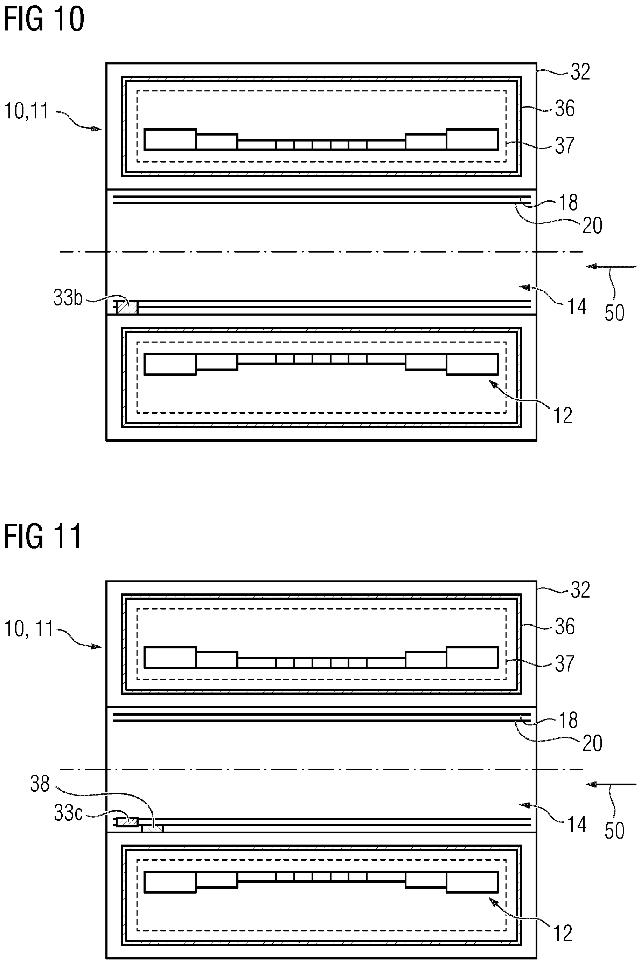

Die Gradientenspule 18 weist vorliegend eine Aussparung zur Aufnahme der Trägerstruktur 33a auf. Es ist vorstellbar, dass die Trägerstruktur 33 lediglich durch die Aussparung in der Gradientenspule 18 durchgreift, um die Körperspule 20 mit der Wand der äußeren Vakuumkammer zu befestigen. Die Gradientenspule 18 kann in diesem Fall separat mittels einer Trägerstruktur 38 (siehe Fig. 10) mechanisch mit der Haltestruktur 32 oder der Wand der äußeren Vakuumkammer verbunden sein.

Die Trägerstruktur 33a kann jedoch auch zu ausgebildet sein, die Körperspule 20 und die Gradientenspule 18 mechanisch mit der Wand der äußeren Vakuumkammer zu verbinden.

Fig. 10 zeigt eine weitere Ausführungsform des erfindungsgemäßen Magnetresonanzgeräts 10. In dem gezeigten Beispiel ist die Trägerstruktur 33b der Körperspule 20 an der Wand der äu-ßeren Vakuumkammer befestigt. Die Trägerstruktur 33b kann hierfür durch eine Aussparung in der Gradientenspule 18 hindurchgreifen und mechanisch mit einem Abschnitt der Wand der Vakuumkammer verbunden sein.

Vorzugsweise ist die Trägerstruktur 33b in einer Aussparung in der Gradientenspule 18 aufgenommen. Die Trägerstruktur 33b kann dazu ausgebildet sein, die Körperspule 20, aber auch die Gradientenspule 18, an der Wand der äußeren Vakuumkammer zu befestigen.

Fig. 11 zeigt eine Ausführungsform des erfindungsgemäßen Magnetresonanzgeräts 10, bei welcher die Körperspule 20 mittels der Trägerstruktur 33c mechanisch mit der Gradientenspule 18 verbunden ist. In dieser Ausführungsform kann die Gradientenspule 18 Aussparungen aufweisen, welche dazu ausgebildet sind, Befestigungsmittel aufzunehmen, welche die Trägerstruktur 33c mit der Gradientenspule 20 verbinden. Die Befestigungsmittel können beispielsweise Schrauben, Bolzen, Stifte, Niete oder dergleichen umfassen.

In dem gezeigten Beispiel weist die Gradientenspule 18 eine Trägerstruktur 38 auf, welches dazu ausgebildet ist, die Gradientenspule 18 mechanisch mit der Wand der äußeren Vakuumkammer zu verbinden. Die Trägerstruktur 38 kann analog zu einer Ausführungsform der Trägerstruktur 33 mit der Wand der äußeren Vakuumkammer verbunden sein. Es ist ebenso vorstellbar, dass die Wand der äußeren Vakuumkammer eine Vertiefung 35 (siehe Fig. 9) aufweist, um einen Abschnitt der Trägerstruktur 38 aufzunehmen oder eine Verankerung der Trägerstruktur 38 in der Vertiefung 35 zu ermöglichen.

Die in Fig. 11 gezeigte Trägerstruktur 33c kann beispielsweise auch als eine provisorische Trägerstruktur ausgestaltet sein. Eine provisorische Trägerstruktur kann dazu ausgebildet sein, die Körperspule 20 reversibel an der Gradientenspule 18 und/oder der Haltestruktur 32 zu befestigen. Die provisorische Trägerstruktur kann insbesondere dazu ausgebildet sein, die Körperspule 20 temporär, z. B. während eines Transports, an der Gradientenspule 18 und/oder der Haltestruktur 32 zu befestigen. Es ist vorstellbar, dass die provisorische Trägerstruktur dazu ausgebildet ist, nach dem Transport entfernt und durch eine konventionelle Trägerstruktur (siehe Fig. 1) ersetzt zu werden.

Obwohl die Erfindung im Detail durch die bevorzugten Ausführungsbeispiele näher illustriert und beschrieben wurde, ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzgerät (10), umfassend eine Haltestruktur (32) und eine Felderzeugungseinheit (11) mit einem Hauptmagneten (12), einem Gradientensystem und einem Hochfrequenzsystem, wobei die Haltestruktur (32) dazu ausgebildet ist, den Hauptmagneten (12) mechanisch zu stützen, und wobei die Felderzeugungseinheit (11) durch ein von der Haltestruktur (32) eingegrenztes Volumen außenumfänglich umschlossen ist.

2. Magnetresonanzgerät (10) nach Anspruch 1, wobei die Felderzeugungseinheit (11) zumindest ein Anschlusselement (34) und/oder eine Trägerstruktur (34,38) umfasst, welche durch das von der Haltestruktur (32) eingegrenzte Volumen außenumfänglich umschlossen ist.

3. Magnetresonanzgerät (10) nach einem der vorhergehenden Ansprüche, wobei das Hochfrequenzsystem eine Trägerstruktur (33) aufweist, welche dazu ausgebildet ist, eine Hochfrequenzspule (20) des Hochfrequenzsystems mechanisch mit der Haltestruktur (32) zu koppeln, wobei die Trägerstruktur (33) durch das von der Haltestruktur (32) eingegrenzte Volumen außenumfänglich umschlossen ist.

4. Magnetresonanzgerät (10) nach Anspruch 3, wobei die Haltestruktur (32) eine äußere Vakuumkammer umfasst, welche den Hauptmagneten (12) außenumfänglich umschließt, wobei eine Wand der äußeren Vakuumkammer eine Vertiefung (35) aufweist und wobei ein Abschnitt der Trägerstruktur (33) des Hochfrequenzsystems zumindest teilweise in der Vertiefung (35) der äußeren Vakuumkammer aufgenommen ist.

5. Magnetresonanzgerät (10) nach Anspruch 1, wobei die Hochfrequenzeinheit eine provisorische Trägerstruktur aufweist, welche dazu ausgebildet ist, eine Hochfrequenzspule (20) des Hochfrequenzsystems reversibel an einer Gradientenspule (18) des Gradientensystems und/oder der Haltestruktur (32) zu befestigen, wobei die provisorische Trägerstruktur reversibel entfernbar ausgestaltet ist und in einer anwendungsgemäßen Anordnung zur Befestigung der Hochfrequenzspule (20) an der Gradientenspule (18) und/oder der Haltestruktur (32) durch das von der Haltestruktur (32) eingegrenzte Volumen außenumfänglich umschlossen ist.

6. Magnetresonanzgerät (10) nach einem der vorhergehenden Ansprüche, wobei eine Hochfrequenzspule (20) des Hochfrequenzsystems ein Anschlusselement (34) aufweist, welches dazu ausgebildet ist, die Hochfrequenzspule (20) mit einer Stromquelle und/oder einem externen Kühlsystem zu verbinden, und wobei das Anschlusselement (34) der Hochfrequenzspule (20) durch das von der Haltestruktur (32) eingegrenzte Volumen außenumfänglich umschlossen ist.

7. Magnetresonanzgerät (10) nach einem der vorhergehenden Ansprüche, wobei die Felderzeugungseinheit (11) ein Anschlusselement (34) aufweist, welches dazu ausgebildet ist, eine Hochfrequenzspule (20) des Hochfrequenzsystems und/oder eine Gradientenspule (18) des Gradientensystem mit einer externen Stromquelle und/oder einem externen Kühlsystem zu verbinden, wobei das Anschlusselement (34) der Felderzeugungseinheit (11) als ein flexibles Verbindungselement ausgestaltet ist und dazu ausgebildet ist, relativ zu dem Hauptmagneten (12) bewegt zu werden und innerhalb des von der Haltestruktur (32) eingegrenzten Volumens verstaut zu werden.

8. Magnetresonanzgerät (10) nach Anspruch 6 oder 7, wobei das Anschlusselement (34) in ein von der Hochfrequenzspule (20) umschlossenes Volumen und/oder einen Patientenaufnahmebereich (14) des Magnetresonanzgeräts (10) hineinragt.

9. Magnetresonanzgerät (10) nach einem der vorhergehenden Ansprüche, wobei eine Gradientenspule (18) des Gradientensystems ein Anschlusselement (34) aufweist, welches dazu ausgebildet ist, die Gradientenspule (18) mit einer Stromquelle und/oder einem externen Kühlsystem zu verbinden, und wobei das Anschlusselement (34) der Gradientenspule (18) durch das von der Haltestruktur (32) eingegrenzte Volumen außenumfänglich umschlossen ist.

10. Magnetresonanzgerät (10) nach Anspruch 9, wobei eine Hochfrequenzspule (20) des Hochfrequenzsystems und/oder die Gradientenspule (18) das Gradientensystem eine Aussparung aufweisen, welche dazu ausgebildet ist, das Anschlusselement (34) der Gradientenspule (18) aufzunehmen.

11. Magnetresonanzgerät (10) nach einem der Ansprüche 9 oder 10, wobei das Anschlusselement (34) der Gradientenspule (18) durch eine Aussparung in einer Hochfrequenzspule (20) des Hochfrequenzsystems hindurchgeführt ist und in ein von der Hochfrequenzspule (20) umschlossenes Volumen und/oder einen Patientenaufnahmebereich (14) des Magnetresonanzgeräts (10) hineinragt.

12. Magnetresonanzgerät (10) nach einem der vorhergehenden Ansprüche, umfassend eine reversibel abnehmbare Anschlussplatte (39), welche dazu ausgebildet ist, eine Gradientenspule (18) des Gradientensystems und/oder eine Hochfrequenzspule (20) des Hochfrequenzsystems mit einer Stromquelle elektrisch und mechanisch zu verbinden.

13. Magnetresonanzgerät (10) nach einem der vorhergehenden Ansprüche, umfassend eine Halterung (31), welche dazu ausgebildet ist, das Magnetresonanzgerät (10) in einem vorbestimmten Abstand zu einer Bodenfläche (71) zu halten, wobei ein Teil der Halterung (31b), welcher eine Abmessung der Haltestruktur (32) in einer Raumrichtung überschreitet, reversibel abnehmbar ausgestaltet ist.

14. Magnetresonanzgerät nach einem der vorhergehenden Ansprüche, umfassend eine Halterung (31), welche dazu ausgebildet ist, das Magnetresonanzgerät (10) in einem vorbestimmten Abstand zu einer Bodenfläche (71) zu halten, wobei die Halterung (31) relativ zu dem Hauptmagneten (12) drehbar und/oder schwenkbar ausgestaltet ist.

15. Magnetresonanzgerät (10) nach einem der vorhergehenden Ansprüche, umfassend eine Außenhülle (30) mit einem reversibel abnehmbaren Abschnitt (30b), wobei eine Benutzerschnittstelle (40), welche von dem abnehmbaren Abschnitt (30b) getragen ist, mittels einer elektrischen Schnittstelle (42) mit einer Steuereinheit (22) des Magnetresonanzgeräts (10) verbunden ist, wobei eine elektrische Anschlussleitung (41), welche die Benutzerschnittstelle (40) mit der elektrischen Schnittstelle (42) verbindet, so ausgestaltet ist, dass ein reversibles abnehmen des abnehmbaren Abschnitts (30b) mit der Benutzerschnittstelle (40) von dem Magnetresonanzgerät (10) ermöglicht wird.

16. Magnetresonanzgerät (10) nach einem der vorhergehenden Ansprüche, umfassend eine Außenhülle (30), wobei ein Abschnitt der Außenhülle (30a) den Hauptmagneten (12) entlang eines Abschnitts einer Patientenzugangsrichtung (50) umschließt und wobei eine Abmessung des Abschnitts der Außenhülle (30a) entlang der Patientenzugangsrichtung (50) eine Abmessung der Haltestruktur (32) entlang der Patientenzugangsrichtung (50) unterschreitet.
